# EUROPEAN PATENT APPLICATION

(11) **EP 2 594 260 A1**
(43) Date of publication of application: **22.05.2013**
(21) Application number: 11189759.1
(22) Date of filing: 18.11.2011
(51) Int. Cl.: A61K 9/28, A61K 31/436

(54) **Solid preparations comprising sirolimus with desired bioavailability and method for its preparation**

(71) Applicant: LEK Pharmaceuticals d.d., 1526 Ljubljana (SI)
(72) Inventor: The designation of the inventor has not yet been filed
(74) Representative: Kunic Tesovic, Barbara

(57) **Abstract**

The present invention relates to a pharmaceutical composition in a solid final dosage form comprising sirolimus exhibiting sufficient bioavailability and therapeutic effect, and a method for preparation thereof. A formulation with suitable therapeutic effect provides benefits in regard of cost reduction, process optimization and reduction in undesired effects regarding the formulation and its behavior *in vivo*

## Description

### Technical Field

The present invention relates to a pharmaceutical composition in a solid final dosage form comprising sirolimus exhibiting sufficient bioavailability and therapeutic effect. The invention further relates to a preferred method for preparation of said solid final dosage form. The pharmaceutical composition comprises sirolimus in particulate form, whereby the particle size of sirolimus particles is in the micro range. Preferably, the pharmaceutical composition does not comprise solubilizers, which are frequently present in the prior art formulations. A formulation with suitable therapeutic effect with high sirolimus particle size without solubilizers provides benefits in regard of cost reduction, process optimization and reduction in undesired effects regarding the formulation and its behavior *in vivo.*

### Background of the Invention

Sirolimus (also known as rapamycin) is a macrolide antibiotic produced by *Streptomyces hygroscopicus* which was discovered first for its properties as an antifungal agent. It adversely affects the growth of fungi such as *Candida albicans* and *Microsporum gypseum.* Sirolimus, its preparation and its antibiotic activity were described in U.S. Pat. No. 3 929 992, issued Dec. 30, 1975 to Surendra Sehgal et al. In 1977 Martel, R. R. et al. 1. reported on immunosuppressive properties of sirolimus against experimental allergic encephalitis and adjuvant arthritis in the Canadian Journal of Physiological Pharmacology, 55, 48-51 (1977). In 1989, Calne, R. Y. et al. in Lancet, 1989, no. 2, p. 227 and Morris, R. E. and Meiser, B. M. in Medicinal Science Research, 1989, No. 17, P. 609-10, separately reported on the effectiveness of sirolimus in inhibiting rejection *in vivo* in allograft transplantation. Numerous articles have followed describing the immunosuppressive and rejection inhibiting properties of sirolimus, and clinical investigations have begun for the use of sirolimus in inhibiting rejection in transplantation in man. Following that, U.S. Pat. No. 5 100 899 was issued which contains description of the use of sirolimus to inhibit transplantation rejection in mammals.

Poor water solubility of sirolimus poses an issue in formulating the drug into suitable dosage form. Many approaches have been used to improve the solubility and dissolution properties of poorly soluble active ingredients including salt formation, particle size reduction, formation of nanoparticles, pH adjustment, use of surfactants, inclusion complexes, use of oily formulations, use of self-emulsifying drug delivery systems, formation of co-precipitates with hydrophilic polymers, co-milling with hydrophilic excipients, and others.

In addition, it has been reported that compositions of sirolimus with conventional excipients can show unpredictable dissolution rates, irregular bioavailability profiles, as well as instability problems. Currently, sirolimus is available in two dosage forms, namely as tablet and oral solution. Sirolimus is dissolved in the solution preparation and the solubility issue has been solved by decreased particle size and the inclusion of solubilizers in the tablet formulation. With decreasing the particle sizes, the surface area increases, which results in higher dissolution rate and improved bioavailability. Surfactants, which act as solubilizing agents, on the other hand, increase the solubility of sirolimus and thus contribute to the increase in the dissolution rate and increased permeability, both aiming at improved bioavailability. Other approaches are based on the idea that the transformation of the crystalline form of a low solubility drug such as sirolimus into the amorphous form can increase the solubility thereof. However, amorphous sirolimus is extremely chemically unstable and is therefore not easily acceptable for the incorporation into an oral pharmaceutical dosage form.

For instance US patent 5 989 591 discloses formulations comprising sirolimus as the active ingredient with a weight average particle size of less than 400 nm while Poloxamer 188 is included in the formulation as solubilizing agent. Further, US patent application 2008/0138405 A1 relates to a solid sirolimus preparation, similar to the preparation described in the US patent 5 989 591, wherein at least 90% of the particles have a volume average particle size of more than about 400 nm, more particularly 400-1200 nm, particularly 400-1000 nm with Poloxamer 407 being included in the formulation as solubilizing agent.

Bioavailability of sirolimus, being a poorly soluble drug, is strongly affected by the particle size and therefore a certain size limit exists above which its bioavailability is too low for the therapeutic effect. Another well-known method for increasing bioavailability of poorly soluble drugs is the inclusion of solubilizing agents in the formulation. When multiple methods for bioavailability increase are used (e.g. size reduction and inclusion of solubilizing agents) a synergistic effect (i.e. greater increase in bioavailability from combination of methods compared to the sum of individual methods) is expected, hence the combination of methods is a common solution for poorly soluble drugs which show poor bioavailability with only one method.

During research work with sirolimus, the present inventors surprisingly found out that certain modifications of the prior art processes and formulations allow for the preparation of sirolimus formulations with high particle size preferably without the inclusion of solubilizing agents, which still exhibit good *in vivo* bioavailability of sirolimus.

Several different solubilizers and binders, including those frequently used in the relevant patent literature, were tested and the difference in their ability to solubilize sirolimus was noted. Finally, final dosage forms comprising sirolimus particles with average particle size greater than 3000 nm without any solubilizers were shown to have higher bioavailability in man compared to the reference product Rapamune®, which has the average particle size below 400 nm and includes poloxamer 188 as solubilizer.

### Summary of the invention

The present invention has been made in the light of the foregoing circumstances and the present invention provides the following items.
1. A solid pharmaceutical composition comprising sirolimus having an average particle size of more than 1 µm, a solubilizer in an amount not more than 15 % relative to the mass content of the active ingredient sirolimus, at least one binder, and optionally pharmaceutically acceptable excipients other than solubilizing agents selected from carriers, fillers, lubricants, glidants, disintegrants, coating agents, preservatives, and coloring material.
2. The composition according to item 1, wherein the sirolimus is crystalline and/or wherein the average particle diameter is 50 µm or less, preferably between 2 to 25 µm, most preferably between 3 to 10 µm.
3. The composition according to item 1 or 2, wherein the composition does not contain any solubilizers.
4. The composition according to any of items 1 to 3, wherein the composition has an active ingredient core, which is preferably coated with a coating layer.
5. The composition according to any of items 1 to 4, wherein the at least one binder is hydroxypropylmethylcellulose or a derivative thereof, and wherein povidone and/or hydroxypropyl cellulose and/or polyethyleneglycol may additionally be used as a binder, and/or wherein a sugar, preferably sucrose, is used as the carrier.
6. The composition according to any of items 1 to 5, wherein the lubricant is sodium stearyl fumarate and/or wherein the glidant is fumed silica and/or wherein the disintegrant / filler is low substituted hydroxypropyl cellulose and/or wherein the coating agent is hydroxypropylmethylcellulose and/or wherein the coloring material is titanium dioxide and/or iron oxide.
7. The composition according to any of items 1 to 6, which is used as a medicament, preferably for the therapy of solid tumors, including sarcomas and carcinomas, such as astrocytomas, prostate cancer, breast cancer, small cell lung cancer, and ovarian cancer; adult T-cell leukemia/lymphoma; fungal infections; hyperproliferative vascular diseases such as restenosis and atherosclerosis; and as immunosuppresive.
8. Method for producing a solid pharmaceutical composition comprising sirolimus, the method comprising the steps:
   (i) Preparing an aqueous dispersion of sirolimus, preferably crystalline sirolimus, together with at least one binder, which is preferably hydroxypropylmethylcellulose or a derivative thereof;
   (ii) Milling the dispersion to obtain a dispersion with particles having an average particle size of more than 1 µm;
   (iv) Spraying the dispersion onto a carrier, preferably a sugar carrier, to obtain a granulate.
9. The method according to item 8, wherein after the step (ii) the method further comprises the step (iii) Admixing water and a carrier, preferably a sugar carrier, optionally a further binder and/or preservatives, to the dispersion.
10. The method according to item 8 or 9, wherein in steps (iii) and (iv) the sugar is sucrose and/or wherein in step (iii) the further binder is povidone and/or the preservative is vitamin E.
11. The method according to any of items 8 to 10, wherein method further comprises the step of
   (v) Compressing the granulate to form an active ingredient core for a tablet, preferably compressing the granulate together with further pharmaceutically acceptable excipients selected from fillers, lubricants, glidants and disintegrants.
12. The method according to item 11, wherein the lubricant is sodium stearyl fumarate and/or wherein the glidant is fumed silica and/or wherein the disintegrant / filler is low substituted hydroxypropyl cellulose.
13. The method according to item 11 or 12, wherein the active ingredient core is coated with an aqueous suspension to form a coat layer, the suspension containing at least one coating agent and optionally coloring material.
14. The method according to item 13, wherein the at least one coating agent of the coat layer is hydroxypropylmethylcellulose or a derivative thereof, wherein preferably hydroxypropylcellulose and/or polyethylene glycol is/are present as further coating agents, and/or wherein the coloring material is titanium dioxide and/or iron oxide.
15. The method according to any of items 8 to 14, wherein the milling of the dispersion to obtain a dispersion in step (ii) is performed to obtain particles having an average particle diameter of 50 µm or less, preferably between 2 to 25 µm, most preferably between 3 to 10 µm.

### Description of the Drawings

Fig. 1 illustrates that solubilizers poloxamer 188 and polysorbate 80 at 1 % (w/v) have a significant influence on the solubility of sirolimus compared to the binders HPMC and PVP.
Fig. 2 shows the analysis results in terms of the dissolution profile of sirolimus by using the tablet according to example 4 and the originator product Rapamune® from the market used as a reference.
Fig. 3 shows the results of the permeability assay of sirolimus in the presence and absence of excipients performed in Caco-2 cell monolayers.

### Detailed Description

According to a first aspect, the present invention relates to a pharmaceutical composition comprising sirolimus having an average particle size of more than 1 µm, a solubilizer in an amount not more than 15 % relative to the mass content of the active ingredient sirolimus, at least one binder, and optionally pharmaceutically acceptable excipients other than solubilizing agents selected from carriers, fillers, lubricants, glidants, disintegrants, coating agents, and coloring material.

Sirolimus according to the invention may be sirolimus per se or a sirolimus derivative or analogue. Sirolimus derivatives are well known in the art and include, for example 32-deoxosirolimus, 16-pent-2-ynyloxy-32-(S)-dihydrosirolimus, 16-O-substituted sirolimus, 26-O-substituted sirolimus, 28-O-substituted sirolimus, 32-O-substituted sirolimus, 40-O-substituted sirolimus, ester derivatives and others. For example, the derivative may be an O-substituted derivative in which the hydroxyl group on the cyclohexyl ring of sirolimus is replaced by -OR1, in which R1 is hydroxyalkyl, hydroxyalkoxyalkyl, acylaminoalkyl and aminoalkyl; for example, 40-O-(2-hydroxy)ethyl-sirolimus, 40-O-(3-hydroxy)propyl-sirolimus, 40-O-[2-(2-hydroxy)ethoxy]ethyl-sirolimus or 40-O-(2-acetaminoethyl)-sirolimus. Preferably, the sirolimus used in the present invention is sirolimus per se.

Sirolimus used in the pharmaceutical compositions of this invention may be in crystalline or amorphous form. Since, however, the amorphous from may pose serious issues, mainly involving physical and chemical stability of the drug as well as possible pharmacokinetic changes in comparison to formulation comprising crystalline particles, crystalline particles are particularly preferred.

The amount of sirolimus in a unit of the pharmaceutical compositions is not specifically restricted and may range from 0.1 to 20 mg, preferably 0.5 to 10 mg, more preferably from 0.5 to 5 mg, most preferably from 0.5 to 2 mg.

The pharmaceutical composition may be in the form of a tablet, a capsule, spheres and granules. In order to attain the best results, preferred is the tablet form, and most preferred a tablet with an active ingredient core.

The present inventors discovered that it is not necessary to provide the sirolimus in the nanosize range in order to exhibit required bioavailability. The inventors found out that sirolimus particles having a large average particle size of above 1 µm exhibit required bioavailability while significantly reducing the milling time of sirolimus. This reduction is directly translated into economic benefits due to time savings and reduced machine wear. Although there is no specific upper limit, it is preferred that the pharmaceutical composition has particles with an average particle size of 50 µm or less in order to attain an acceptable bioavailability. Preferably, the average particle size is in the range of 2 to 25 µm, and most preferably between 3 and 10 µm in order to attain the best results according to the invention.

Further, the present inventors surprisingly found that contrary to the prior art teaching it is not necessary to add solubilizers in significant amount in order to attain good bioavailability *in vivo* even when using the sirolimus with the large average particle size. Therefore, according to the invention, the pharmaceutical composition contains not more than 15 % by weight of solubilizer, preferably not more than 5 % by weight, relative to the mass content of the active ingredient sirolimus. Solubilizers according to the invention are defined as solubilizing agents that exhibit a solubility enhancement index (SI) above 30. The solubility enhancement index (SI) is defined as the ratio of the drug concentration after 24 hours incubation period in the excipient (solubilizing agent) present media versus the solubility of sirolimus at 24 hours incubation period in phosphate buffer pH 6.8 at 1 % (w/v). Typical solubilizers know in the art comprise compounds or certain derivatives thereof such as polyethoxylated fatty acids, PEG-fatty acid diesters, polyethylene glycol glycerol fatty acid esters, alcohol-oil transesterification products, polyglycerized fatty acids, propylene glycol fatty acid esters, mono- and diglycerides, sterol and sterol derivatives, polyethylene glycol sorbitan fatty acid esters, polyethylene glycol alkyl ethers, sugar esters, polyethylene glycol alkyl phenols, polyoxyethylene-polyoxypropylene block copolymers, sorbitan fatty acid esters, lower alcohol fatty acid esters, derivatives of fat-soluble vitamins and ionic surfactants (e.g. sodium dodecylsulfate).

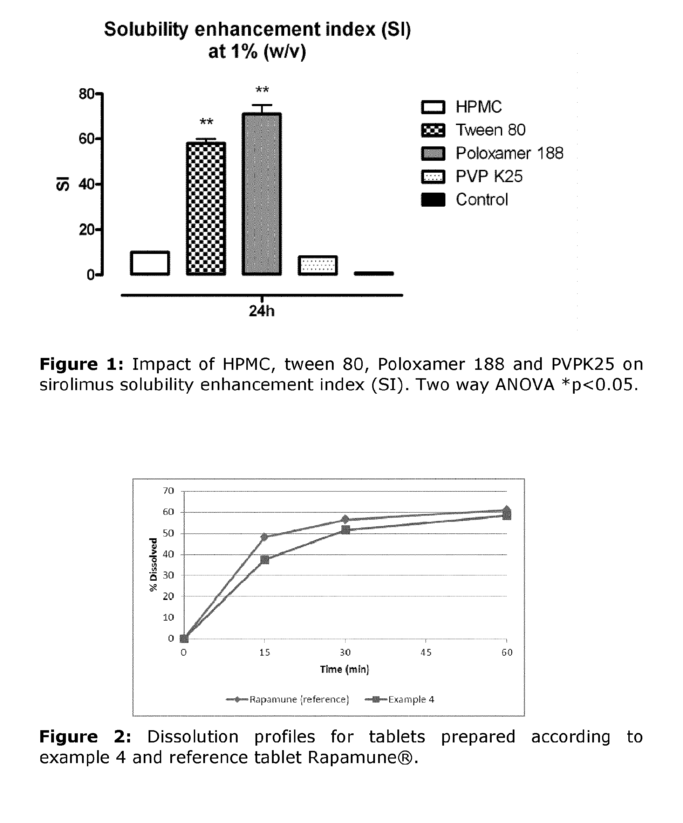

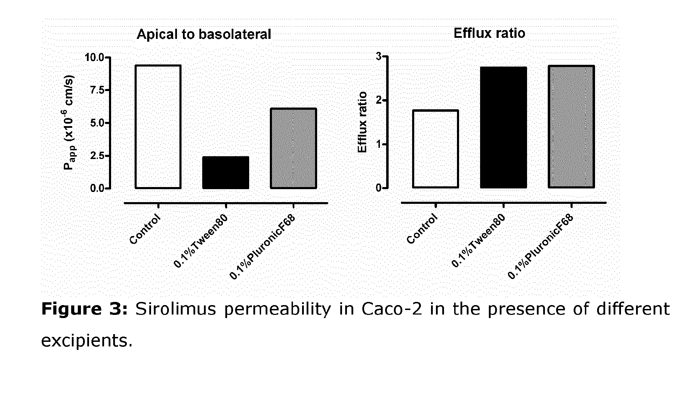

Surprisingly, the inventors found out that although the SI of above 50 for the solubilizer Tween 80 and Poloxamer 188 (Pluronic *F 68*) (see Fig. 1) indicate a significantly improved water solubility of sirolimus, Caco-2 cell layer experiments indicate an undesired action of these solubilizers as illustrated in Fig. 3 (Example 7).

Therefore, according to the most preferred embodiment, solubilizers are excluded from the pharmaceutical composition of the present invention. The omission of solubilizers can directly reduce the complexity of the formulation and reduce the production costs with the further benefit of excluding all potential unwanted interactions of solubilizing agents with the drug or other excipients in the formulation and unwanted *in vivo* interactions of solubilizing agents.

The pharmaceutical composition comprises at least one binder. Typical binders which can be used in the pharmaceutical composition of the present invention include methyl cellulose, hydroxypropyl cellulose, hydroxylpropylmethycellulose, polyvinylpyrrolidone, gelatin, gum Arabic, ethyl cellulose, polyvinyl alcohol, pullulan, pregelatinized starch, agar, tragacanth, carboxymethyl cellulose, sodium alginate, propylene glycol, microcrystalline cellulose or mixtures thereof.

Preferably, the pharmaceutical composition contains at least hydroxypropylmethylcellulose (HPMC) or a derivative thereof as binder which has impact on the good bioavailability of the pharmaceutical composition. More preferably, the HPMC binder is contained in the active ingredient core if the pharmaceutical composition is provided in the form of a tablet. In this case, the HPMC binder in the active ingredient core is preferably contained with 0.1 to 2 mg per unit of the pharmaceutical composition, preferably 0.25 to 1 mg, most preferably between 0.4 to 0.75 mg in order to attain the best effects.

The pharmaceutical composition may contain further binder in addition to the HMPC binder. This further binder is preferably low molecular povidone (PVP 25). The addition amount of the further (PVP) binder may range from 0.2 to 2, more preferably, 0.5 to 1 mg per unit of the pharmaceutical composition. Most preferably, this additional PVP binder is present in the active ingredient core of the pharmaceutical composition if provided as a tablet.

Yet further, the pharmaceutical composition preferably further comprises a carrier. The carrier is preferably a sugar carrier including lactose, mannitol, sorbitol, sucrose and mixtures thereof. In the pharmaceutical composition of the present invention, preferably sucrose is used for achieving the best results. The amount of the carrier is not specifically limited. However, it is preferred that the carrier may range from 50 to 500 mg per unit of the pharmaceutical composition, more preferably from 100 to 250 mg, most preferably from 150 to 200 mg.

Preferably, the carrier is provided in the active ingredient core of a tablet together with the HPMC binder, most preferably together with the HPMC and the PVP binder.

The pharmaceutical composition may preferably contain preservatives such as antioxidants commonly known. While the amount is not specifically limited, it preferably ranges from 0.05 to 1 mg per unit of the pharmaceutical composition, more preferably from 0.1 to 0.5 mg. The pharmaceutical composition of the present invention preferably contains vitamin E as preservative. If provided in the preferred form of a tablet with an active ingredient core, the preservative is preferably contained in the core of the tablet.

The pharmaceutical composition preferably comprises pharmaceutically acceptable excipients other than solubilizing agents selected from fillers, lubricants, glidants, disintegrants, coating agents, and coloring material.

As lubricants, commonly known agents may be used in the present invention including sodium stearyl fumarate, stearic acid, magnesium stearate, calcium stearate, hydrogenated castor oil, and mixtures thereof. The amount is not specifically restricted. Preferably, the amount ranges from 0.1 to 10 wt.-% per unit of the pharmaceutical composition, more preferably 0.5 to 5 wt.-%, most preferably from 0.8 to 1.5 wt.-%.

In the pharmaceutical composition of the invention, preferably sodium stearyl fumarate is used as lubricant.

As glidants, commonly known agents may be used in the present invention, such as fumed silica, talc, and magnesium carbonate.

The amount is not specifically restricted. Preferably, the amount ranges from 0.2 to 8 wt.-% per unit of the pharmaceutical composition, more preferably from 0.5 to 5 wt.-%, most preferably from 0.5 to 1 wt.-%.

In the pharmaceutical composition of the invention, preferably fumed silica (Aerosil 200) is used as glidant.

As disintegrants, commonly known agents may be used in the present invention, such as starches or modified starches, croscarmellose sodium, low substituted hydroxypropyl cellulose, and sodium starch glycolate and mixtures thereof.

The amount of the disintegrant is not specifically restricted. Preferably, the amount ranges from 2 to 40 mg per unit of the pharmaceutical composition, more preferably from 5 to 30 mg, most preferably from 10 to 20 mg.

In the pharmaceutical composition of the invention, preferably at least low substituted hydroxypropyl cellulose is used as disintegrant.

Also fillers may be present in the pharmaceutical composition of the present invention. These fillers are used to fill out the size of e.g. the tablet or capsule for adjusting a suitable dosage from if desired. The filler should be inert, compatible with the other components of the formulation, non-hygroscopic, relatively cheap, and preferably tasteless or pleasant tasting. Known fillers such as for instance plant cellulose (pure plant filler), dibasic calcium phosphate, lactose, glucose, mannitol, sorbitol, xylane, calcium carbonate, and magnesium stearate can be used as fillers in the composition of the present invention. The amount of fillers in a unit of the pharmaceutical composition of the present invention is not specifically restricted, but may preferably range from 2-25 wt.-%, more preferably 5-15 wt.-%.

If provided in the preferred form of a tablet having an active ingredient core, the above lubricants, fillers, glidants, and disintegrants are preferably provided in the tablet core.

In the case of the pharmaceutical composition being a tablet having an active ingredient core, the tablet core is preferably coated with a coat. As coating agents conventional fillers/binder agents may be used. The amount of the coating agents is not specifically restricted. Preferably, the amount ranges from 2 to 30 mg per unit of the pharmaceutical composition, more preferably from 4 to 20, most preferably from 5 to 10 mg.

Preferably, at least HPMC or a derivative thereof is used as coating agent. Still more preferably, hydroxypropylcellulose and/or polyethylene glycol (400) may be used as coating agent in addition to HPMC. The HPMC is preferably the major component among the coating agents and preferably ranges from 1 to 20, more preferably 2 to 10, most preferably 3 to 7 mg per unit of the pharmaceutical composition.

The pharmaceutical composition may also contain coloring material. The coloring material is not specifically restricted as long as it is pharmaceutically acceptable and non-limiting examples include titanium dioxide, quinolone yellow and iron oxides. In a preferred formulation, the pharmaceutical composition contains titanium dioxide and iron oxides such as iron oxide brown and yellow (E172). The amount of the coloring material is not specifically restricted. It may preferably range from 0.02 to 6 mg per unit of the pharmaceutical composition, more preferably from 0.1 to 3 mg, most preferably from 0.5 to 2 mg for the coloring material or a mixture thereof.

Preferably, the coloring material is provided in the coat layer if the pharmaceutical composition is provided in the form of a tablet preferably having an active ingredient core.

In a preferred embodiment, the pharmaceutical composition essentially consists or even consists of the excipient defined by claim 1. More preferably, the pharmaceutical composition comprises the excipients defined by the examples of the present invention. Even more preferably, the pharmaceutical composition of the present invention may essentially consist or consist of the excipients illustrated by the examples without being restricted to the specific ranges defined by those examples.

According to a further aspect, the present invention provides a method for producing a solid pharmaceutical composition comprising sirolimus. The method is a particularly preferred method for producing a favorable pharmaceutical composition according to the invention.

According to one aspect, the method for producing a pharmaceutical composition comprises the steps:
(i) Preparing an aqueous dispersion of sirolimus, preferably crystalline sirolimus, together with at least one binder;
(ii) Milling the dispersion to obtain a dispersion with particles having an average particle size of more than 1 µm;
(iv) Spraying the dispersion onto a carrier, preferably a sugar carrier, to obtain a granulate.

The at least one binder is defined as described above. Preferably, the at least one binder is hydroxypropylmethylcellulose or a derivative thereof, more preferably in the above defined ranges. If hydroxypropylmethylcellulose is contained as the at least one binder, the invention can implement the best results concerning the bioavailability of the pharmaceutical composition although the particle size is more than 1 µm and although solubilizers are not present or present in an amount not more than 15 % relative to the mass content of the active ingredient sirolimus.

In the most preferred method according to the invention, no solubilizers are used in the method for manufacturing the pharmaceutical composition.

As described above, it is preferred that the pharmaceutical composition is milled to attain particles with an average particle size of 50 µm or less in order to attain an acceptable bioavailability. More preferably, the milling is performed to provide particles having an average particle size in the range of 2 to 25 µm, and most preferably between 3 and 10 µm in order to attain the best results according to the invention.

Any conventional milling methods may be applied in step (ii) of the above method. Suitable methods include ball mill, a vibratory mill, and media mills. Preferably, a ball mill is used for economically milling the suspension to achieve the desired micro scale particles.

The suspension in step (iv) is sprayed on a carrier, wherein the carrier may be the same as previously described. Preferred is the use of sugar carriers, more preferably in the above defined ranges. The sugar carrier used during manufacturing of the present invention may be a sugar as defined above, and is preferably sucrose.

Commonly know spraying methods may be used to give the resultant granulate. In the present invention, a fluid bed apparatus turned out to be particularly preferred.

Preferably, the method contains a further step (iii) after the step (ii) of admixing water and a carrier, preferably a sugar carrier, optionally a binder and/or preservatives, to the dispersion before the spraying step (iv).

The sugar carrier may be selected among the above defined compounds and is preferably the same sugar as used in step (iv) and is most preferably sucrose. Further binders as the ones defined above may additionally be admixed in step (iii). According to a preferred embodiment, povidone (PVP 25) is added as a further binder in step (iii) in addition to the sugar carrier.

Also preservatives may preferably be added in the step (iii). The amounts and kinds of preservatives used during manufacturing are preferably the same as defined above.

The method preferably further comprises the step of (v) compressing the granulate to form an active ingredient core for a tablet. More preferably, the compressing of the granulate is accomplished together with further pharmaceutically acceptable excipients selected from fillers, lubricants, glidants, and disintegrants.

The further pharmaceutically acceptable excipients selected from fillers, lubricants, glidants and disintegrants and the respective amounts are preferably the same as previously described. In a preferred embodiment, the manufacturing method comprises a step (v) in which fillers, lubricants, glidants, and disintegrants are added when compressing the granulate obtained in step (iv). It is more preferred that the lubricant is sodium stearyl fumarate and/or wherein the glidant is fumed silica (e.g. aerosil 200) and/or wherein the disintegrant / filler is low substituted hydroxypropyl cellulose. Particularly preferred, the step (v) uses sodium stearyl fumarate, fumed silica (e.g. aerosil 200), and low substituted hydroxypropyl cellulose in the above ranges when compression the granulate to attain most favorably attributes.

The method of the present invention may further contain a step where the active ingredient core is coated with an aqueous suspension to form a coat layer, the suspension for providing the coating containing at least one coating agent and optionally coloring material. Usual coating techniques may be applied in this method step such as spray coating in a conventional coating pan or fluidized bed processor, or dip coating. The coating agent(s) and the amount(s) thereof is/are preferably the same as defined above.

When applying such a coating step, the at least one coating agent of the coat layer is preferably hydroxypropylmethylcellulose. It is still more preferred to add further coating agents selected from preferably hydroxypropylcellulose and/or polyethylene glycol (polyethylene glycol 400).

When adding coloring material during the coating, the coating material(s) and the amount(s) thereof is/are preferably the same as defined above. In a most preferred embodiment, titanium dioxide and iron oxides (brown and yellow) are used in the coating step.

Also a seal coating can be applied for protection of the active ingredient core.

The pharmaceutical composition of the present invention is preferably used as a medicament. Basically, the new pharmaceutical composition of the present invention is not limited to a specific therapy but encompasses all diseased which can be treated by the administration of a composition contained sirolimus as active ingredient.

Non-limiting examples for preferred therapies include the treatment of cancer, in particular of solid tumors, including sarcomas and carcinomas, such as astrocytomas, prostate cancer, breast cancer, small cell lung cancer, and ovarian cancer; adult T-cell leukemia / lymphoma. Also fungal infections and hyperproliferative vascular diseases such as restenosis and atherosclerosis may be treated by the pharmaceutical composition. The pharmaceutical composition may preferably used as immunosuppressive, for instance to inhibit transplantation rejection.

The present invention provides a new pharmaceutical composition and a method for its production, which attains such outstanding effects as having a high bioavailability even after oral application, while significantly reducing the resources for its production and simplifying its formulation. By completely omitting the presence of solubilizers, potential side effects and unwanted *in vivo* actions etc. do not occur, while the inventors found a surprising way to maintain or even improve the *in vivo* bioavailability of the scarcely soluble drug sirolimus by means of the new pharmaceutical composition and the way of its production. As such, it turned out that the provision of the pharmaceutical composition in the form of a tablet containing an active ingredient core contributes to attain such effects, while also the presence of the binder HPMC has great benefit in attaining the outstanding effect.

### Examples

As used herein, particle size is measured by particle size measuring techniques well known to those skilled in the art. Such techniques include sedimentation field flow fractionation, photon correlation spectroscopy, laser light scattering, such as using a Malvern Mastersizer (Malvern Instruments Ltd., United Kingdom), and disk centrifugation. "Average particle size" as used herein refers to at least 90% of the drug particles having weight average particle sizes below the stated particle size measured by Malvern Mastersizer.

As used herein, suspension is a suspension of sirolimus in water together with a suitable stabilizer.

### EXAMPLE 1:

Excipient solutions of hydroxypropyl methylcellulose (HPMC), polyvinylpyrrolidone (PVP), polysorbate 80 and poloxamer 188 were prepared by dissolving the appropriate excipient in phosphate buffer pH 6.8. Sirolimus was dissolved in DMSO and dispensed into each well of the polypropylene microtiter plate (MTP), followed by 50-times dilution with excipient solutions in phosphate buffer pH 6.8. MTP were immediately sealed followed by incubation (37 °C, 550 rpm). The final DMSO content in the tested media was 2 % (v/v). Samples were taken after 24 hour incubation, centrifuged, and analyzed by UPLC.

Influence of the excipients was tested in regard to the pure sirolimus diluted with phosphate buffer pH 6.8 instead of excipient solution in the same way as described above. The solubility enhancement index (SI) was defined as the ratio of the drug concentration after 24 hours in the excipient present media versus the solubility of sirolimus at 24 hours in phosphate buffer pH 6.8 at 1 % (w/v). As evident from Figure 1, solubilizers poloxamer 188 and polysorbate 80 at 1 % (w/v) have a significant influence on sirolimus solubility compared to binders HPMC and PVP.

### EXAMPLE 2:

### Tablet composition:

| **Excipient chemical name** | **Excipient common name (brand name)** | **Amount per unit (mg)** |
|---|---|---|
| Sirolimus | Sirolimus | 2.00 |
| Cellulose hydroxypropyl methyl ether | HPMC, Hypromellose | 0.50 |
| β-d-fructofuranosyl-α-d-glucopyranoside | Sucrose | 174.75 |
| (±)-(2RS,4'RS,8'RS)-2,5,7,8-Tetramethyl-2-(4',8',12'-trimethyltridecyl)-6-chromanol | Vitamin E | 0.25 |
| 2-Butenedioic acid, monooctadecyl ester, sodium salt | Sodium stearyl fumarate | 1.50 |
| Silica | Fumed silica (Aerosil 200) | 1.00 |
| Cellulose, 2-hydroxypropyl ether (low-substituted) | Hydroxypropyl cellulose, low substituted | 15.00 |
| Titanium dioxide | Titanium dioxide | 1.00 |
| Iron oxide, Yellow (E172) | Iron oxide, Yellow (E172) | 0.06 |
| Iron oxide, Brown (E172) | Iron oxide, Brown (E172) | 0.04 |
| Cellulose hydroxypropyl methyl ether | Hypromellose | 4.00 |
| Cellulose, 2-hydroxypropyl ether | Hydroxypropyl cellulose | 1.00 |
| α-Hydro-ω-hydroxypoly(oxy-1,2-ethanediyl) | Polyethylene Glycol 400 | 0.90 |

### Manufacturing procedure:

HPMC is dissolved in water and sirolimus is dispersed into the solution. The resulting suspension is milled by a ball mill to obtain a suspension of sirolimus particles in the desired size range. Part of sucrose is mixed with vitamin E dissolved in ethanol and ethanol is removed. The suspension is further mixed with water and sucrose with vitamin E. The resulting suspension is sprayed onto the remaining sucrose in a fluid bed apparatus. Dried granulate is mixed with sodium stearyl fumarate, colloidal silicon dioxide and low substituted hydroxypropyl cellulose and compressed into tablet cores.

Hypromellose used as coating agent, hydroxypropyl cellulose and polyethylene glycol 400 are dissolved in water, titanium dioxide and iron oxides are added. The cores are coated with the resulting suspension.

### EXAMPLE 3:

### Tablet composition:

| **Excipient chemical name** | **Excipient common name (brand name)** | **Amount per unit (mg)** |
|---|---|---|
| Sirolimus | Sirolimus | 2.00 |
| Cellulose hydroxypropyl methyl ether | HPMC, Hypromellose | 0.50 |
| β-d-fructofuranosyl-α-d-glucopyranoside | Sucrose | 175.00 |
| 2-Butenedioic acid, monooctadecyl ester, sodium salt | Sodium stearyl fumarate | 1.50 |
| Silica | Fumed silica (Aerosil 200) | 1.00 |
| Cellulose, 2-hydroxypropyl ether (low-substituted) | Hydroxypropyl cellulose, low substituted | 15.00 |
| Titanium dioxide | Titanium dioxide | 1.00 |
| Iron oxide, Yellow (E172) | Iron oxide, Yellow (E172) | 0.06 |
| Iron oxide, Brown (E172) | Iron oxide, Brown (E172) | 0.04 |
| Cellulose hydroxypropyl methyl ether | Hypromellose | 4.00 |
| Cellulose, 2-hydroxypropyl ether | Hydroxypropyl cellulose | 1.00 |
| α-Hydro-ω-hydroxypoly(oxy-1,2-ethanediyl) | Polyethylene Glycol 400 | 0.90 |

### Manufacturing procedure:

HPMC is dissolved in water and sirolimus is dispersed into the solution. The resulting suspension is milled by a ball mill to obtain a suspension of sirolimus particles in the desired size range. The suspension is further mixed with water and a part of sucrose. The resulting suspension is sprayed onto the remaining sucrose in a fluid bed apparatus. Dried granulate is mixed with sodium stearyl fumarate, colloidal silicon dioxide and low substituted hydroxypropyl cellulose and compressed into tablet cores.

Hypromellose used as coating agent, hydroxypropyl cellulose and polyethylene glycol 400 are dissolved in water, titanium dioxide and iron oxides are added. The cores are coated with the resulting suspension.

### EXAMPLE 4:

### Tablet composition:

| **Excipient chemical name** | **Excipient common name (brand name)** | **Amount per unit (mg)** |
|---|---|---|
| Sirolimus | Sirolimus | 2.00 |
| Cellulose hydroxypropyl methyl ether | HPMC, Hypromellose | 0.50 |
| β-d-fructofuranosyl-α-d-glucopyranoside | Sucrose | 174.25 |
| 1-Ethenyl-2-pyrrolidinone homopolymer | Povidone (PVP K25) | 0.75 |
| 2-Butenedioic acid, monooctadecyl ester, sodium salt | Sodium stearyl fumarate | 1.50 |
| Silica | Fumed silica (Aerosil 200) | 1.00 |
| Cellulose, 2-hydroxypropyl ether (low-substituted) | Hydroxypropyl cellulose, low substituted | 15.00 |
| Titanium dioxide | Titanium dioxide | 1.00 |
| Iron oxide, Yellow (E172) | Iron oxide, Yellow (E172) | 0.06 |
| Iron oxide, Brown (E172) | Iron oxide, Brown (E172) | 0.04 |
| Cellulose hydroxypropyl methyl ether | Hypromellose | 4.00 |
| Cellulose, 2-hydroxypropyl ether | Hydroxypropyl cellulose | 1.00 |
| α-Hydro-ω-hydroxypoly(oxy-1,2-ethanediyl) | Polyethylene Glycol 400 | 0.90 |

### Manufacturing procedure:

HPMC is dissolved in water and sirolimus is dispersed into the solution. The resulting suspension is milled by a ball mill to obtain a suspension of sirolimus particles in the desired size range. The suspension is further mixed with water, povidone and a part of sucrose. The resulting suspension is sprayed onto the remaining sucrose in a fluid bed apparatus. Dried granulate is mixed with sodium stearyl fumarate, colloidal silicon dioxide and low substituted hydroxypropyl cellulose and compressed into tablet cores.

Hypromellose used as coating agent, hydroxypropyl cellulose and polyethylene glycol 400 are dissolved in water, titanium dioxide and iron oxides are added. The cores are coated with the resulting suspension.

The average particle size of the all the above sirolimus suspensions after milling measured by a laser diffraction method (Malvern Mastersizer) was 3.3 µm.

### EXAMPLE 5:

Tablets prepared according to example 4 and the originator product Rapamune® from the market used as a reference was analyzed in terms of the dissolution profile of sirolimus. Samples were analyzed on an USP Apparatus 2 (paddles) with stationary baskets (20 mesh) in 500 mL FaSSIF with pH 6.5 and are shown on Figure 2. FaSSIF is widely recognized in the scientific literature as a biorelevant dissolution medium, which mimics the dissolution behavior *in vivo*.

Considering the differences in the dissolution profiles one might consider that increased dissolution of the reference compared to the example 4 is due to the presence of surfactants in the reference formulation. Further, one might expect higher bioavailability *in vivo.*

### EXAMPLE 6:

Tablets prepared according to example 4 were given to healthy fasted human volunteers and the originator product Rapamune® from the market was used as a reference. After ingestion blood samples were collected at predetermined intervals and the concentration of sirolimus was determined. Key pharmacokinetic (PK) parameters (AUC and Cmax) were calculated for both samples tested and the ratio of the least squares means (LS Means) were determined.

**Table 1: Comparison of PK parameters of tablets prepared according to example 5 against marketed reference Rapamune®.**

| Statistical analysis (ANOVA) | Treatment comparison | Ratio of LS means |
|---|---|---|
| AUC_{0-72 h} | Sirolimus Example 4 vs Rapamune® (reference) | 116.01 % |
| Cₘₐₓ | Sirolimus Example 4 vs Rapamune® (reference) | 166.79 % |

Despite the relatively high particle size of the Active Pharmaceutical Ingredients (API) sirolimus in example 4, the absence of surfactants acting as solubilizers in the formulations and consequently the slower dissolution *in vitro*, the behavior of tablets prepared according to example 4 showed better bioavailability *in vivo* compared to the reference tablet Rapamune®, which is a tablet having an inert core being coated with an active coat comprising poloxamer solubilizer and nanosized sirolimus.

### EXAMPLE 7:

Prior to use in the experiment, Caco-2 cell monolayers were grown to confluence on collagen coated, microporous, polycarbonate membranes in 12-well Costar TranswellR plates. The permeability assay of sirolimus in the presence and absence of excipients was performed in Caco-2 cell monolayers. The results are shown in Figure 3. An influence of solubilizers such as surfactants on the permeability of sirolimus in CaCo-2 can be observed, indicating a possible undesired *in vivo* interaction, which could lower the bioavailability of sirolimus.

## Claims

1. A solid pharmaceutical composition comprising sirolimus having an average particle size of more than 1 µm, a solubilizer in an amount not more than 15 % relative to the mass content of the active ingredient sirolimus, at least one binder, and optionally pharmaceutically acceptable excipients other than solubilizing agents selected from carriers, fillers, lubricants, glidants, disintegrants, coating agents, preservatives, and coloring material.

2. The composition according to claim 1, wherein the sirolimus is crystalline and/or wherein the average particle diameter is 50 µm or less, preferably between 2 to 25 µm, most preferably between 3 to 10 µm.

3. The composition according to claim 1 or 2, wherein the composition does not contain any solubilizers.

4. The composition according to any of claims 1 to 3, wherein the composition has an active ingredient core, which is preferably coated with a coating layer.

5. The composition according to any of claims 1 to 4, wherein the at least one binder is hydroxypropylmethylcellulose or a derivative thereof, and wherein povidone and/or hydroxypropyl cellulose and/or polyethyleneglycol may additionally be used as a binder, and/or wherein a sugar, preferably sucrose, is used as the carrier.

6. The composition according to any of claims 1 to 5, wherein the lubricant is sodium stearyl fumarate and/or wherein the glidant is fumed silica and/or wherein the disintegrant / filler is low substituted hydroxypropyl cellulose and/or wherein the coating agent is hydroxypropylmethylcellulose and/or wherein the coloring material is titanium dioxide and/or iron oxide.

7. The composition according to any of claims 1 to 6, which is used as a medicament, preferably for the therapy of solid tumors, including sarcomas and carcinomas, such as astrocytomas, prostate cancer, breast cancer, small cell lung cancer, and ovarian cancer; adult T-cell leukemia / lymphoma; fungal infections; hyperproliferative vascular diseases such as restenosis and atherosclerosis; and as immunosuppresive.

8. Method for producing a solid pharmaceutical composition comprising sirolimus, the method comprising the steps:
(i) Preparing an aqueous dispersion of sirolimus, preferably crystalline sirolimus, together with at least one binder, which is preferably hydroxypropylmethylcellulose or a derivative thereof;
(ii) Milling the dispersion to obtain a dispersion with particles having an average particle size of more than 1 µm;
(iv) Spraying the dispersion onto a carrier, preferably a sugar carrier, to obtain a granulate.

9. The method according to claim 8, wherein after the step (ii) the method further comprises the step (iii) Admixing water and a carrier, preferably a sugar carrier, optionally a further binder and/or preservatives, to the dispersion.

10. The method according to claim 8 or 9, wherein in steps (iii) and (iv) the sugar is sucrose and/or wherein in step (iii) the further binder is povidone and/or the preservative is vitamin E.

11. The method according to any of claims 8 to 10, wherein method further comprises the step of
(v) Compressing the granulate to form an active ingredient core for a tablet, preferably compressing the granulate together with further pharmaceutically acceptable excipients selected from fillers, lubricants, glidants and disintegrants.

12. The method according to claim 11, wherein the lubricant is sodium stearyl fumarate and/or wherein the glidant is fumed silica and/or wherein the disintegrant / filler is low substituted hydroxypropyl cellulose.

13. The method according to claim 11 or 12, wherein the active ingredient core is coated with an aqueous suspension to form a coat layer, the suspension containing at least one coating agent and optionally coloring material.

14. The method according to claim 13, wherein the at least one coating agent of the coat layer is hydroxypropylmethylcellulose or a derivative thereof, wherein preferably hydroxypropylcellulose and/or polyethylene glycol is/are present as further coating agents, and/or wherein the coloring material is titanium dioxide and/or iron oxide.

15. The method according to any of claims 8 to 14, wherein the milling of the dispersion to obtain a dispersion in step (ii) is performed to obtain particles having an average particle diameter of 50 µm or less, preferably between 2 to 25 µm, most preferably between 3 to 10 µm.
